# EUROPÄISCHE PATENTSCHRIFT

(11) **EP 1 549 949 B1**
(45) Veröffentlichungstag und Bekanntmachung des Hinweises auf die Patenterteilung: **17.02.2010**
(21) Anmeldenummer: 03756478.8
(22) Anmeldetag: 08.10.2003
(51) Int. Cl.: G01N 33/50

(54) **VERFAHREN ZUR ERKENNUNG UND EINGRENZUNG DES RISIKOS FÜR BRUSTKREBS DURCH DEN MIKRONUKLEUSTEST AN ZWEIKERNIGEN ZELLEN**
METHOD FOR DETECTING AND LIMITING BREAST CARCINOMA RISK BY A MICRONUCLEUS TEST OF BINUCLEAR CELLS
PROCEDE POUR DEPISTER ET LIMITER LE RISQUE DE CANCER DU SEIN PAR UN TEST DU MICRONUCLEUS EFFECTUE SUR DES CELLULES A DEUX NOYAUX

(30) Priorität: 08.10.2002 DE 10246811
(43) Veröffentlichungstag der Anmeldung: 06.07.2005
(73) Patentinhaber: Vogel, Walter, 89081 Ulm (DE)
(72) Erfinder: Vogel, Walter, 89081 Ulm (DE)
(74) Vertreter: Schnappauf, Georg
(86) Internationale Anmeldenummer: PCT/EP2003/011141
(87) Internationale Veröffentlichungsnummer: WO 2004/034055

(56) Entgegenhaltungen:
- WO-A-02/14859
- SCOTT D ET AL: "Radiation-induced micronucleus induction in lymphocytes identifies a high frequency of radiosensitive cases among breast cancer patients: A test for predisposition?" BRITISH JOURNAL OF CANCER, Bd. 77, Nr. 4, Februar 1998 (1998-02), Seiten 614-620, XP009026905 ISSN: 0007-0920 in der Anmeldung erwähnt
- ROTHFUSS ANDREAS ET AL: "Induced micronucleus frequencies in peripheral lymphocytes as a screening test for carriers of a BRCA1 mutation in breast cancer families" CANCER RESEARCH, Bd. 60, Nr. 2, 15. Januar 2000 (2000-01-15), Seiten 390-394, XP001179734 ISSN: 0008-5472
- TRENZ KRISTINA ET AL: "Mutagen sensitivity of peripheral blood from women carrying a BRCA1 or BRCA2 mutation" MUTATION RESEARCH, Bd. 500, Nr. 1-2, 20. März 2002 (2002-03-20), Seiten 89-96, XP001179736 ISSN: 0027-5107
- FENECH M ET AL: "MEASUREMENT OF MICRONUCLEI IN LYMPHOCYTES" MUTATION RESEARCH, Bd. 147, Nr. 1-2, 1985, Seiten 29-36, XP009027290 ISSN: 0027-5107

## Beschreibung

### Gebiet der Erfindung

Die vorliegende Erfindung betrifft ein Verfahren zur Früherkennung und Eingrenzung des Risikos für Brustkrebs bei einem Testindividuum durch den Mikronukleustest an induzierten zweikernigen Zellen ohne Induktion durch Mutagen-Behandlung der kultivieren Zellen des Testindividuums.

### Hintergrund der Erfindung

Die Früherkennung von Brustkrebs stellt trotz der jüngsten Entwicklung von Vorhersageverfahren ('t Veer et al. 2002, Nature 415:530-536) noch immer ein Problem der Diagnostik und Prognostik dar.

Mit einem Anteil von etwa 31 % aller Krebserkrankungen bei Frauen ist Brustkrebs die häufigsten Krebserkrankungen bei Frauen (Greenlee et al. 2001, CA: Cancer J. Clin. 51:15-36). Nach Lungenkrebs ist Brustkrebs darüber hinaus mit 15 % die zweithäufigste Todesursache bei Krebserkrankungen überhaupt (Greenlee et al. 2001, CA: Cancer J. Clin. 51:15-36).

Jedoch ist aufgrund der vielfältigen Ursachen, die zur Ausbildung von Brustkrebs führen können, eine sichere Abschätzung des Risikos einer Erkrankung bisher nur für kleinere Untergruppen möglich (Scott et al. 1998, Br. J. Cancer 77:614-620; Scott et al. 1999, Int. J. Radiat. Biol. 75:1-10). Als Ursachen einer Brustkrebserkrankung sind genetische Faktoren (wie z. B. Mutationen im BRCA1 bzw. BRCA2-Gen) bekannt oder es werden Umweltfaktoren (wie Exposition gegenüber mutagenen Substanzen oder Strahlung) vermutet.

Mutationen in den BRCA-Genen, BRCA1 und BRCA2, sind die Hauptursache bei erblichem oder familiärem Brustkrebs, d.h. bei gehäuft auftretenden Erkrankungen innerhalb einer Familie. Bei Frauen, die nicht einer Risikofamilie angehören, tragen diese Mutationen aber nur zu etwa 2 % zu den Ursachen einer Brustkrebserkrankung bei (Peto et al. 1999, J. Natl. Cancer Inst. 91:943-949). Etwa 10% aller Fälle von Brustkrebs sind erblich, d.h. dem familären Brustkrebs zuzurechnen. Für andere Betroffene, die nicht zu einer bestimmten Brustkrebs-Risikofamilie gehören (nichtfamiliäre, sporadische Brustkrebsfälle) ist selbst mit den modernen Verfahren, z. B. durch Bestimmung von Expressionsprofilen durch Mikroarray-Analyse, keine Risikoabschätzung vor Erkennung des Tumors möglich, da die auslösenden Gene nicht bekannt sind und ihr Expressionsprofil erst im Tumor selbst messbar wäre.

Etwa 90% aller Brustkrebsfälle stellen Einzelfälle innerhalb der Familie dar (sporadische Brustkrebsfälle), deren Ursache bislang noch nicht bekannt sind. Vermutet wird ein Zusammenwirken vieler verschiedene Faktoren, von der Ernährung über Geburtenzahl und Stillzeiten, bis hin zu einem Zusammenwirken von Effekten verschiedener noch unbekannter Gene.

Der Bestimmung des Brustkrebsrisikos, das auf einer Mutation in BRCA1 oder BRCA2 basiert, ist mit den modernen Verfahren sehr verlässlich möglich. Im Gegensatz dazu ist die Früherkennung oder die Bestimmung des Brustkrebsrisikos aus Niedrigrisikofamilien, d. h. aus Familien, deren Brustkrebs nicht auf eine BRCA-Mutation zurückzuführen ist, oder aus Familien mit sporadischem Auftreten von Brustkrebs mit den bekannten Verfahren nur sehr ungenau möglich.

Es ist bekannt, dass bei Erkrankungen in Hochrisikofamilien, also in Familien mit einer Mutation in einem der BRCA-Gene, eine erhöhte Sensitivität gegenüber einer Mutagen-Behandlung besteht (Rothfuss et al. 2000, Cancer Res. 60:390-394; Trenz et al. 2002, Mutation Res. 500:89-96).

BRCA1 und BRCA2 sind, neben anderen Funktionen, auch an der Aufrechterhaltung der genomischen Integrität beteiligt (Trenz et al. 2002, *supra*; Venkitaraman 2001, J. Cell Sci. 114:3591-3598; Welcsh et al. 2000, Trends Genet. 16:69-74). Beide Proteine sind Teil eines Multiproteinkomplexes, der an der Doppelstrangbruch-Reparatur und der homologen Rekombination beteiligt ist. Daneben finden sich in diesem Komplex weitere Proteine, die aus anderen Reparaturwegen bekannt sind (Venkitaraman 2002, Cell 108: 171-182). Gene, die diese Komponenten kodieren, scheinen zum Teil auch als Risikofaktor für Krebs in Frage zu kommen, z. B.LIG4 (O'Driscoll et al. 2001, Mol. Cell 8:1175-1185).

Die Messung des Effektes von Mutagenen wurde bisher verwendet, um z. B. die Kanzerogenität von neuen Pharmazeutika oder kanzerogene Umwelteinflüsse zu untersuchen (WO 02/14859). Einer der hierfür geeigneten Tests ist der sogenannte Mikronukleustest (MNT). Er misst induzierte Chromosomenaherrationen in einer Zellkultur von proliferierenden Zellen, die auch aufgrund einer gestörten DNA-Doppelstrangreparatur nur unvollständig behoben werden können (Berwick und Vineis 2000, J. Natl. Cancer Inst. 92:1536-1537). Der MNT (Mikronukleustest) ist ein Standardverfahren des Biomonitoring bzw. der Mutagenitätstestung (z.B. Kirsch-Volders et al. 2000, Environ. Mol. Mutagen. 35:167-172).

Rothfuss Andreas et. al., Cancer Research, Bd 60. Nr. 2, 15. Januar 2000,1 Seiten 390-394 und Trenz Kristina et. al., Mutation Research, Bd. 500, Nr. 1-2, 20. März 2002, Seiten 89-96, offenbaren die Verwendung der Messung der induzierten Mikronuklei-Rate für die Ermittlung des Brustkrebsrisikos von familiären Brustkrebspatienten.
Fenech M. et al., Mutation Research, Bd. 147, Nr- 1-2, 1985, Seiten 29-36 offenbart die Anwendung des Mikronuleus - Tests auf Lymphozyten zur Bestimmung von mutagenen Agenzien.

Hinsichtlich der Ermittlung des Brustkrebsrisikos wurde die Sensitivität gegenüber Mutagenen für Mutationsträger in BRCA1 und BRCA2 (Rothfuss ct al. 2000, *supra*; Trenz et al. 2002, *supra*) genutzt. Hinsichtlich des sporadischen Brustkrebses oder von Brustkrebs in Niedrigrisikofamilien wurden zwei Studien durchgeführt. Hier konnte jedoch in den untersuchten Fällen nicht generell ein Unterschied zwischen Betroffenen und Kontrollen festgestellt werden (Scott et al. 1998, *supra*; Scott et al. 1999, *supra*). Vielmehr wurde je nach Ausführung des MNT nur für verschiedene Untergruppen (entsprechend etwa 30% der Betroffenen) eine signifikant erhöhte Mikronukleusrate nachgewiesen.

Es existiert somit kein Verfahren zur Bestimmung und Eingrenzung des Risikos für Brustkrebs bei Frauen, die keine Träger einer BRCA-Mutation sind, d. h. in Niedrigrisikofamilien bzw. für sporadische Brustkrebsfälle.

Sporadischer Brustkrebs tritt unabhängig von der genetischen Prädisposition als Einzelfall innerhalb der Familie auf und ist unabhängig von der Zugehörigkeit der Probandin zu einer bestimmten Brustkrebs-Risikofamilie .(wie z.B. eine Mutation im BRCA1- oder BRCA2-Gen).

Somit ist es eine Aufgabe der vorliegenden Erfindung, ein Verfahren bereitzustellen, mit dessen Hilfe das Brustkrebsrisiko in allen Fällen genau und sicher bestimmt werden kann. Bevorzugt soll mit dem erfindungsgemäßen Verfahren das Hrustkrebsrisiko für sporadische Brustkrebsfälle bestimmt werden. Die Verwendung des Verfahrens für sporadische Brustkrebsfälle ermöglicht es, das Brustkrebsrisiko in den Fällen genau und sicher zu bestimmen, in denen die Frau nicht aus einer Risikofamilie stammt.

### Zusammenfassung der Erfindung

Das Problem der Bestimmung und Eingrenzung des Risikos für Brustkrebs in Niedrigrisikofamilien oder für sporadischen Brustkrebs wird mit dem erfindungsgemäßen Verfahren gelöst. Die Lösung ergibt sich aus den Patentansprüchen, der nachfolgenden Beschreibung und den Abbildungen.

Die vorliegende Erfindung stellt ein Verfahren zur Früherkennung und Eingrenzung des Risikos von Brustkrebs, bei einem Testindividuum durch die Verwendung einer modifizierten Variante des Miloronukleustests zur Verfügung. Hierbei wird die spontane Bildung von Mikronuklei in zweikernigen (binukleären) Zellen bestimmt. Die Risikoabschätzung erfolgt durch den. Vergleich der ermittelten Mikronukleusraten mit Kontrollgruppen gesunder und erkrankter Testindividuen. Der Vergleich erfolgt durch allgemein übliche statistische Verfahren und liefert ein Maß, um wie viel höher das Risiko einer Brustkrebserkrankung im Vergleich zu einer Kontrollgruppe gesunder Individuen liegt.

### Detaillierte Beschreibung der Erfindung

Die vorliegende Erfindung betrifft ein Verfahren zur Bestimmung und Eingrenzung des Risikos für Brustkrebs durch den Milaonukleustest an binukleären Zellen. Das Verfahren zur Risikobestimmung für sporadischen Brustkrebs bietet gegenüber der bisherigen Situation den Vorteil, dass jedem Testindividuum unabhängig von der Familienvorgeschichte eine Untersuchung angeboten werden kann, die eine genauere Bestimmung des Brustkrebsnsikos ermöglicht.

In der vorliegenden Erfindung wurde überraschend festgestellt, dass Probanden mit sporadischem Brustkrebs sich durch eine erhöhte Sensitivität gegenüber Röntgenstrahlung und anderen bekannten Mutagenen regelmäßig charakterisieren lassen: Eine solche Sensitivität war wissenschaftlich nicht zu erwarten. Mit Hilfe des erfindungsgemäßen Verfahrens lassen sich das nicht familiäre sporadische Brustkrebsrisiko bei Probanden ermitteln.

Das erfindungsgemäße Verfahren umfasst in einer Ausführungsform die folgenden Schritte: (1) Aus Heparin-behandeltem Blut werden Lymphozyten gewonnen und kultiviert, (2) die Lymphozytenkultur wird mit einem Mitogen zur Induktion des Zellzyklus stimuliert, und (3) die Zellteilung wird 40-50 Stunden nach Anlegen der Kultur mit einem Zytokinese-Inhibitor unterbunden, nicht jedoch die Kernteilung. Schließlich wird (4) die Kultur ca. 12-24 Stunden nach Zugabe des Zytokinese-Inhibitors abgebrochen und fixiert, (5) zytologische Präparate hieraus hergestellt und (6) die Mikronukleusrate bestimmt Aus der Mikronukleusrate wird (7) das Risiko der Erkrankung an Brustkrebs im Vergleich zu Kontrollgruppen ermttelt.

### Definitionen

Unter "Brustkrebs" ist das Auftreten eines bösartigen Tumors in der Brustdrüse zu verstehen. Bei Frauen ist dies mit etwa 31 % eine der häufigsten malignen Erkrankungen überhaupt (Greenlee et al. 2001, *supra*) und sehr viel häufiger als bei Männern, die meist Träger einer Mutation im BRCA2 Gen sind und einer Hochrisikofamilie angehören.

Unter "Brustkrebsrisiko" ist die Wahrscheinlichkeit für das Auftreten von Brustkrebs zu verstehen.

Unter "sporadischem Brustkrebsrisiko" ist das Risiko zur Brustkrebserkrankung unabhängig von der Zugehörigkeit der Probanden zu einer Brustkrebs-Risikofamilie zu verstehen. Als Beispiel ist das Auftreten einer Brustkrebserkrankung als Einzelfall bei mehreren blutsverwandten Personen innerhalb einer Familie zu verstehen.

Unter "familiärem Brustkrebs" ist das Auftreten von Brustkrebs bei mehreren blutsverwandten Personen innerhalb einer Familie zu verstehen. Eine solche familiäre Häufung der Erkrankung kann durch zufälliges zusammentreffen mehrerer Einzelfälle ohne gemeinsame Ursache zustande kommen, oder auf eine gemeinsame (genetische) Ursache zurückzuführen sein. Diese genetischen Ursachen werden allgemein als zwei Kategorien zugehörig angesehen:
(1) genetische Risikofaktoren mit geringer Wirksamkeit (Penetranz) und vergleichsweise großer Häufigkeit in der Bevölkerung, die in Familien mit wenigen (zwei Betroffene Verwandte ersten Grades) Erkrankten angenommen werden.
(2) Viele (≥ 2) Betroffene und/oder ein früher Erkrankungsbeginn (bei Frauen vor der Menopause) machen eine genetische Ursache mit hoher Wirksamkeit (Penetranz) wahrscheinlich (siehe zum Beispiel die Empfehlungen der Deutschen Krebshilfe). Diese Familien werden als "Hochrisikofamilien" bezeichnet. In etwa zwei Drittel dieser Familien lassen sich Mutationen (funktionszerstörende Veränderung der Basensequenz des Gens) in den BRCA1 bzw. BRCA2 Genen als Ursache der Erkrankung nachweisen. In den anderen Hochrisikofamilien werden Mutationen in bislang unbekannten Genen als Ursache angenommen. Genetisch bedingter Brustkrebs kann nur dann mit Sicherheit angenommen werden, wenn eine Mutation in einem der BRCA Gene nachgewiesen ist.

Ein "Mitogen" im Sinne der Erfindung ist eine chemische Substanz, die die Fähigkeit besitzt, Zellen aus Zellkulturen zur Kernteilung anzuregen.

Ein "Mutagen" ist eine chemische Substanz oder eine physikalische Strahlung, die in der Lage ist, genetisches Material in seiner Struktur zu verändern. Die Veränderung des genetischen Materials kann die Erzeugung von chromosomalen Aberrationen, z. B. von Mikronuklei, sein.

Unter einem "Zytokinese-Inhibitor" wird eine chemische Substanz verstanden; die den Prozess der Zellteilung, d. h. der Segregation der Zytoplasmas einer Zelle auf die beiden Tochterzellen hemmt.

"Induziert" wird im erfindungsgemäßen Sinn so verstanden, als durch die Einwirkung von einem Mutagen gebildet.

Ein "Mikronukleus" wird als genetisches Material, das aufgrund von Chromosomendefekten außerhalb der Zellkerns existiert, verstanden.

"Mikronukleusrate" bedeutet die Anzahl zweikerniger (binukleärer) Zellen, in denen spontan oder durch die Einwirkung eines Mutagens ein Mikronukleus gebildet wird. Die Mikronukleusrate wird im erfindungsgemäßen Sinn als Anzahl von Zellen mit Mikronuklei bezogen auf 1000 binukleäre Zellen dargestellt.

Die "Spontanrate" oder "spontane Mikronukleusrate" ist die Anzahl zweikerniger (binukleärer) Zellen, in denen spontan, d. h. ohne Einwirkung eines Mutagens, ein Mikronukleus gebildet wird. Die Mikronukleusrate wird im erfindungsgemäßen Sinn als Anzahl von Zellen mit Mikronuklei bezogen auf 1000 binukleäre Zellen dargestellt.

Die "induzierte Mikronukleusrate" bedeutet die Anzahl von zweikernigen (binukleären) Zellen, in denen aufgrund der Behandlung mit einem Mutagen ein Mikronukleus gebildet wird. Die induzierte Mikronukleusrate wird im erfindungsgemäßen Sinn als Anzahl von Zellen mit durch ein Mutagen induzierten Mikronuklei bezogen auf 1000 binukleäre Zellen dargestellt.

Unter "peripherem. Blut" wird ein biphasisches Flüssigkeitssystem verstanden, das aus zellulären Bestandteilen in einem flüssigen Plasma besteht. Die zellulären Bestandteile umfassen Erythrozyten, Leukozyten und Thrombozyten, die flüssige Plasmaphase besteht aus Wasser, in dem Proteine gelöst sind.

Unter "Lymphozyten" wird ein bestimmter Zelltyp der Leukozyten verstanden, der im Lichtmikroskop keine granuläre Struktur des Zytplasmas aufweist Lymphozyten besitzen eine Größe zwischen 7 und 18 µm. Der Zellkern läßt sich mit Giemsalösung stark blauviolett färben.

In einer Ausführungsform umfasst der Mikronukleustest die folgenden Schritte: (1) Aus Heparin-behandeltem peripherem Blut werden Lymphozyten gewonnen und kultiviert, (2) die Lymphozytenkultur wird mit einem Mitogen zur Induktion des Zellzyklus stimuliert, und (3) die Zellteilung wird mit Zytokinese-Inhibitoren unterbunden, nicht jedoch die Kernteilung. Schließlich wird (4) die Kultur abgebrochen und fixiert, (5) zytologische Präparate hieraus hergestellt und (6) die Mikronukleusrate bestimmt Aus der spontanen Mikronukleusrate wird (7) das Risiko der Erkrankung an Brustkrebs im Vergleich zu Kontrollgruppen ermittelt

Zusammen mit dem erfindungsgemäßen Verfahren kann ein Test verwendet werden, in welchem eine Mutagen-Behandlung der Lymphozytenkultur sofort bis eine Stunde nach Anlage der Kultur durchgeführt wird. Das Verfahren umfasst die folgenden Schritte: (1) Aus Heparin-behandeltem peripherem Blut werden Lymphozyten gewonnen und kultiviert, und (2) mit einem Mutagen behandelt (3) Die Lymphozytenkultur wird mit einem Mitogen zur Induktion des Zellzyklus stimuliert, und (4) die Zellteilung wird mit einem Zytokinese-Inhibitor unterbunden, nicht jedoch die Kernteilung. Schließlich wird (5) die Kultur abgebrochen und fixiert, (6) zytologische Präparate hieraus hergestellt und (7) die Mikronukleusrate bestimmt. Durch den Vergleich der induzierten Mikronukleusrate mit der induzierten Mikronukleusrate in Kontrollgruppen wird (8) das Risiko der Erkrankung an Brustkrebs ermittelt.

Aush verwendet werden kann ein Test, in welchem eine Mutagen-Behandlung sofort bis eine Stunde nach Anlage der Kultur durchgeführt wird. Das Verfahren wird wie vorstehend beschrieben durchgeführt, jedoch wird die Mikronukleusrate von binukleären Zellen mit zwei oder mehr Mikronuklei bestimmt Durch den Vergleich der Rate des Auftretens von zwei oder mehr Mikronuklei in binukleären Zellen mit der Rate des Auftretens von zwei oder mehr Mikronuklei in binukleären Zellen in Kontrollgruppen wird schließlich das Risiko einer Erkrankung an Brustkrebs ermittelt.

In einer bevorzugten Ausführungsform der erfindungsgemäßen Verfahrens, wie vorstehend beschrieben, werden die ermittelten Mikronukleusraten miteinander kombiniert, um die Sicherheit der Eingrenzung des Risikos, an Brustkrebs zu erkranken, weiter zu erhöhen.

Als Kontrollgruppen werden Probanden ohne Brustkrebs sowie Patienten mit nachgewiesener Brustkrebserkrankung herangezogen. Die Kontrollgruppe der Brustkrebserkrankten setzt sich sowohl aus BRCA-Mutationstcägern sowie aus Patienten mit sporadischem Brustkrebs zusammen. Nach Möglichkeit wird der Anteil der BRCA-Mutationsträger, d. h. der Erkrankten aus Hochrisikofamilien, möglichst gering gehalten.

Gegenüber der bisher verwendeten Verfahren besitzt das erfindungsgemäße Verfahren entscheidende Vorteile. Die Ermittlung des Bruatkrebsrisikos beschränkt sich nicht nur auf die Hochrisikogruppen BRCA1- und BRCA2-Mutationsträger, sondern ist für alle Patientinnen ohne Einschränkung anwendbar.

Außerdem wird gemäß dem Verfahren der vorliegenden Erfindung erstmals die Spontanrate der Mikronukleusbildung und die Rate von zwei oder mehr Mikronuklei in binukleären Zellen zur Ermittlung des Risikos einer Brustkrebserkrankung verwendet Darüber hinaus ist die Ermittlung des Brustkrebsrisikos durch die Kombination der verschiedenen Mikronukleusraten im Vergleich zu bisherigen Verfahren mit erhöhter Sicherheit möglich.

Die Durchführung des erfindungsgemäßen Verfahrens erfolgt in einer bevorzugten Ausführungsform wie nachstehend beschrieben: Es wird eine Lymphozyten-Zellkultur aus heparinisiertem peripheren Blut angelegt Methoden zur Anlage von Lymphozyten-Zellkulturen sind dem Fachmann bekannt.

Gemäß der vorliegenden Erfindung werden die Lymphozyten des peripheren Blutes in Chromosommedium 1A (Gibco-BRL) bei 37 °C inkubiert.

Innerhalb der ersten 6 Stunden nach Anlage der Zellkultur wird zur Stimulation der Kernteilung der kultivierten Zellen ein Mitogen zugesetzt. Vorzugsweise erfolgt die mitogene Stimulation bereits innerhalb der ersten 30 Minuten nach Anlage der Kultur. Besonders bevorzugt ist die Zugabe des Mitogens bei der Anlage der Kultur. Bevorzugte Mitogene sind Leukoagglutinin (PHA-L), Phytohämagglutinin M, Lipopolysaccharid (LPS), Concanavalin A (ConA) oder *Phytolacca americana*-Mitogene (Pokeweed-Mitogen, PWM). Als besonders bevorzugte Mitogene werden Phytohämagglutinin M und Leukoagglutinin (PHA-L) verwendet. Insbesondere bevorzugt ist Phytohämagglutinin M.

Nach 40-50 Stunden nach Anlegen der Kultur erfolgt die Zugabe eines Zytokinese-Inhibitors, bevor die kultivierten Zellen die erste Mitose durchlaufen. Die Zugabe eines Zytokinese-Inhibitors, einer Substanz, die, ohne die Kernteilung zu beeinträchtigen, die Zellteilung verhindert, bewirkt die Entstehung von binukleären Zellen.

Vorzugsweise werden Cytochalasine als Zytokinese-Inhibitoren verwendet. Besonders bevorzugt bei Lymphozytenkulturen ist die Verwendung von Cytochalasin B. Die Zugabe des Zytokinese-Inhibitors erfolgt vorzugsweise nach 44-55 Stunden nach Anlage der Zellkultur. Besonders bevorzugt ist die Zugabe von Cytochalasin B nach 44 Stunden nach der Anlage der Zellkultur in einer Konzentration von 6 µg/ml. Die Kultur wird vorzugsweise für weitere 12-24 Stunden bei 37 °C weiter kultiviert. Besonders bevorzugt ist die weitere Kultivierung der Lymphozytenkultur für 20-24 Stunden.

Nach 12-24 Stunden nach der Zugabe des Zytokinese-Inhibitors wird die Zellkultur vorzugsweise durch Zentrifugation bei 1000 Umdrehungen pro Minute (UpM) für 10 Minuten und milder hypotoner Behandlung mit 0,56 % KCl abgebrochen und fixiert. Die Fixierung erfolgt vorzugsweise durch Waschen der Zellen mit einer Fixierlösung aus Methanol/Eisessig (5:1) und einem gleichen Anteil 0,9 % NaCl. Anschließend werden die Zellen vorzugsweise dreimal mit einer weiteren Fixierlösung bestehend aus Methanol/Eisessig (5:1) gewaschen. Eine fixierte Zellsuspension wird erhalten.

Aus dieser fixierten Zellsuspension werden zytologische Präparate hergestellt. Hierzu werden vorzugsweise Zellen aus der fixierten Suspension auf Objektträger aufgetropft und luftgetrocknet.

Nachfolgend erfolgt die Bestimmung der Mikronukleusrate. Vorzugsweise werden dazu die zytologischen Präparate vor der Auszählung der Zellen gefärbt. Als Farbstoff bevorzugt ist ein Farbstoff, der entweder selektiv den Nukleus oder Nukleus und Zytoplasma differentiell anfärbt. Besonders bevorzugt ist ein Farbstoff ausgewählt aus der Gruppe bestehend aus Giemsa, Acridinorange, 4',6'-Diamino-2-phenylindol (DAPI) oder Hoechst 33258.

Die Giemsafärbung wird mit einer 3%igen-10%igen, vorzugsweise mit einer 5%igen, gepufferten Giemsalösung durchgeführt. Acridinorange wird vorzugsweise in einer Konzentration von 10 µg/ml eingesetzt, DAPI in einer Konzentration von 1 µg/ml. Anschließend wird im Mikroskop, je nach Färbung im Durchlicht oder bei Fluoreszenzbeleuchtung, typischerweise die Anzahl Mikronuklei bezogen auf 1000 zweikernige Zellen durch Beobachtung und Auszählen bestimmt. Mit Giemsa gefärbte Zellen können im Durchlichtmikroskop ausgewertet, d. h. ausgezählt werden. Mit DAPI, Acridinorange oder Hoechst 33258 gefärbte Zellen können in einem Fluoreszenzmikroskop ausgewertet werden. Die Auszählung erfolgt manuell, mit Hilfe von automatischer Bildanalyse am Mikroskop oder mit Hilfe von Durchflußzytometrie. Bevorzugt ist die Auszählung mit Hilfe von automatischer Bildanalyse.

Die Bestimmung von mikronukleus-enthaltenden Erythrozyten mittels Durchflußzytometrie ist im US Patent 6,100,038 beschrieben.

Die Risikoermittlung erfolgt durch den Vergleich der spontanen Mikronukleusrate der Probandin mit der spontanen Mikronukleusrate in Kontrollgruppen. Der Vergleich erfolgt durch allgemein übliche statistische Verfahren und liefert ein Maß, um wieviel höher das Risiko einer Brustkrebserkrankung im Vergleich zu einer Kontrollgruppe gesunder Individuen liegt.

Auch kann ein Verfahren zur Erkennung von Brustkrebs verwendet werden (nicht beansprucht), bei dem unmittelbar bis eine Stunde nach Anlegen der Zellkultur eine Mutagenbehandlung erfolgt: (1) Anlage einer Zellkultur, (2) Behandlung der Zellkultur mit einem Mutagen, (3) mitogene Stimulation der Zellkultur, (4) Zugabe eines Zytokinese-Inhibitors, (5) Abbruch der Kultur, (6) Herstellung eines zytologischen Präparates, (7) Bestimmung der Mikroaukleusrate und (8) Risikoermittlung für das Auftreten von Brustkrebs durch Ermittlung der Mikronukleusrate in binukleären Zellen.

Vorzugsweise wird das Verfahren wie folgt durchgeführt:
Eine Lymphozyten-Zellkultur aus heparinisiertem peripheren Blut wird angelegt. Methoden zur Anlage von Lymphozyten-Zellkulturen sind dem Fachmann bekannt.

Die Lymphozyten des peripheren Blutes werden vorzugsweise in Chromosommedium 1A (Gibco-BRL) bei 37 °C inkubiert.

Sofort bis eine Stunde nach Anlegen der Lymphozytenkultur wird diese mit einem Mutagen behandelt. Vorzugsweise erfolgt die Mutagen-Behandlung mit dem Anlegen der Kultur.

Durch die Verwendung eines Mutagens kann die Rate der gebildeten Mikronuklei erhöht werden. Das Verfahren basiert auf der Beobachtung, dass mit einem erhöhten Krebsrisiko die Mikronukleusrate, die durch eine Mutagen-Behandlung induziert wird, ebenfalls ansteigt. Als Mutagen können chemische Substanzen oder physikalische Strahlung verwendet werden. Als chemische Mutagene können alle Substanzen verwendet werden, die dem Fachmann als mutagenisierende Substanzen bekannt sind. Bevorzugte ist die Verwendung von Wasserstoffperoxid. Wasserstoffperoxid wird bevorzugt in einer Konzentration von 1-5 mM verwendet, insbesondere bevorzugt ist eine Konzentration von 2 mM.

Mutagenisierende physikalische Strahlungen sind vorzugsweise energiereiche Strahlungen. Bevorzugt sind Strahlungen wie Röntgenstrahlung oder Gammastrahlung. Insbesondere bevorzugt ist die Verwendung von Gammastrahlung. Gammastrahlung wird in einer Dosis von 0,5-4 J/kg (Gy), bevorzugt von 2 J/kg (Gy) verwendet.

Als Strahlenquelle kann jede beliebige Strahlenquelle verwendet werden, die Strahlung der erforderlichen Dosis erzeugt. Bevorzugt ist die Verwendung einer Co-60-Strahlenquelle. Besonders bevorzugt ist eine Cs-137-Strahlenquelle.

Innerhalb von 0-6 Stunden nach Anlage der Zellkultur wird zur Stimulation der Kernteilung der kultivierten Zellen ein Mitogen zugesetzt. Vorzugsweise erfolgt die mitogene Stimulation bereits innerhalb der ersten 30 Minuten nach Anlage der Kultur. Besonders bevorzugt ist die Zugabe des Mitogens bei der Anlage der Kultur.

Bevorzugte Mitogene sind Leukoagglutinin (PHA-L), Phytohämagglutinin M. Lipopolysaccharid (LPS), Concanavalin A (ConA) oder *Phytolacca americana*-Mitogene (Pokeweed-Mitogen, PWM). Als besonders bevorzugte Mitogene werden Phytohämagglutinin M und Leukoagglutinin (PHA-L) verwendet. Insbesondere bevorzugt ist Phytohänaagglutinin M.

Nach 40-50 Stunden nach Anlegen der Kultur erfolgt die Zugabe eines Zytokinese-Inhibitors, bevor die kultivierten Zellen die erste Mitose durchlaufen. Die Zugabe eines Zytokinese-Inhibitors bewirkt die Entstehung von binukleären Zellen.

Vorzuasweise werden Cytochalasine als Zytokinese-Inhibitoren verwendet. Besonders bevorzugt bei Lymphozytenkulturen ist die Verwendung von Cytochalasin B. Die Zugabe des Zytokinese-Inhibitors erfolgt vorzugsweise nach 40-55 Stunden nach Anlage der Zellkultur. Besonders bevorzugt ist die Zugabe von Cytochalasin B nach 44 Stunden nach der Anlage der Zellkultur in einer Konzentration von 6 µg/ml. Die Kultur wird vorzugsweise für weitere 12-24 Stunden bei 37 °C weiter kultiviert. Besonders bevorzugt ist die weitere Kultivierung der Lymphozytenkultur für 20-24 Stunden.

Nach 12-24 Stunden nach der Zugabe des Zytokinese-Inhibitors wird die Zellkultur vorzugsweise durch Zentrifugation bei 1000 Umdrehungen pro Minute (UpM) für 10 Minuten und milder hypotoner Behandlung mit 0,56 % KCl abgebrochen und fixiert. Die Fixierung erfolgt vorzugsweise durch Waschen der Zellen mit einer Fixierlösung aus Methanol/Eisessig (5:1) und einem gleichen Anteil 0,9 % NaCl. Anschließend werden die Zellen vorzugsweise dreimal mit einer weiteren Fixierlösung bestehend aus Methanol/Eisessig (5:1) gewaschen. Eine fixierte Zellsuspension wird erhalten.

Aus dieser fixierten Zellsuspension werden zytologische Präparate hergestellt. Hierzu werden vorzugsweise Zellen aus der fixierten Suspension auf Objektträger aufgetropft und luftgetrocknet.

Nachfolgend erfolgt die Bestimmung der Mikronukleusrate. Vorzugsweise werden dazu die zytologischen Präparate vor der Auszählung der Zellen gefärbt. Als Farbstoff bevorzugt ist ein Farbstoff, der entweder selektiv den Nukleus oder Nukleus und Zytoplasma differentiell anfärbt. Besonders bevorzugt ist ein Farbstoff ausgewählt aus der Gruppe bestehend aus Giemsa, Acridinorange, 4',6'-Diamino-2-phenylindol (DAPI) oder Hoechst 33258.

Die Giemsafärbung wird mit einer 3%igen-10%igen, vorzugsweise mit einer 5%igen, gepufferten Giemsalösung durchgeführt. Acridinorange wird vorzugsweise in einer Konzentration von 10 µg/ml eingesetzt, DAPI in einer Konzentration von 1 µg/ml.

Anschließend wird im Mikroskop, je nach Färbung im Durchlicht oder bei Fluoreszenzbeleuchtung, die induzierte Mikronukleusrate durch Beobachtung und Auszählen bestimmt. Mit Giemsa gefärbte Zellen können im Durchlichtmikroskop ausgewertet, d. h. ausgezählt werden. Mit DAPI, Acridinorange oder Hoechst 33258 gefärbte Zellen können in einem Fluoreszenzmikroskop ausgewertet werden. Die Auszählung erfolgt manuell, mit Hilfe von automatischer Bildanalyse am Mikroskop oder mit Hilfe von Durchflusszytometrie. Bevorzugt ist die Auszählung mit Hilfe von automatischer Bildanalyse.

Die Bestimmung von mikronukleus-enthaltenden Erythrozyten mittels Durchflusszytometrie ist im US Patent 6,100,038 beschrieben.

Die induzierten Mikronukleusrate wird bestimmt und für die Risikoermittlung einer Brustkrebserkrankung herangezogen. Die Risikoermittlung für Brustkrebs erfolgt durch den Vergleich der induzierten Mikronukleusrate in binukleären Zellen einer Probandin mit der induzierten Mikronukleusrate in binukleären Zellen in Kontrollgruppen.

Auch verwendet wird ein Verfahren das die Rate des Auftretens von zwei bzw. zwei und mehr Mikronuklei in binukleären Zellen ermittelt Daraus wird das Risiko einer Brustkrebserkrankung im Vergleich zur Rate des Auftretens von zwei bzw. zwei und mehr Mikronuklei in binukleären Zellen in Kontrollgruppen ermittelt.

In einer bevorzugten Ausführungsform des erfindungsgemäßen Verfahrens werden zur Ermittlung des Risikos für Brustkrebs die induzierte Mikronukleusrate in binukleären Zellen, die Spontanrate von Mikronuklei in binukleären Zellen und der Anzahl von binukleären Zellen mit zwei oder zwei und mehr Mikronuklei kombiniert. Daraus kann das Risiko für die Erkrankung an Brustkrebs für eine größere Zahl von Individuen mit höhere Sicherheit vorherbestimmt werden.

Für die Familien, die den Kriterien einer Hochrisikofamilie nicht genügen und für die nichtfamiliären (sporadischen) Fälle von Brustkrebs und deren Blutsverwandte gibt es zur RisikoAbschätzung derzeit keine Verfahren, außer Risikoabscbätzungen auf der Basis der Familienvorgeschichte (Anamnese) und von Bevölkerungsstatistiken.

Die Kombination verschiedener Mikronukleusraten erlaubt es nun, das Risiko für die Erkrankung an sporadischem Brustkrebs oder aufgrund von genetischen Risikofaktoren mit geringer Penetranz verhältnismäßig sicher zu bestimmen. Die Risikobestimmung ergibt sich dann aus der relativen Lage der individuell ermittelten Mikronukleusraten bezogen auf eine Kontrollgruppe. Typischerweise lässt sich diese relative Lage der individuellen Mikronukleusrate als relatives Risiko ("Odds Radio") ausdrucken, daneben gibt es eine große Zahl weiterer statistischer Verfahren, um den gleichen Sachverhalt auszudrücken, die im Grunde alle gleichermaßen hierfür geeignet sind.

Der Schwellenwert, ab dem das Risiko einer Brustkrebserkrankungangenommen wird, wird z.B. wie nachstehend beschrieben, ermittelt: Mikronukleustests werden an Zellen von gesunden und an Brustkrebs erkrankten Personen durchgeführt. Für jede der beiden Gruppen wird die Verteilung der Werte erstellt und Perzentilen (der Prozentsatz von Probanden unterhalb des jeweiligen Wertes) ermittelt, bevorzugt bei einer Mikronukleusrate, unterhalb der 75 % der Mikronukleuaraten gesunder Kontrollpersonen liegen, wird der Schwellenwert für ein erhöhtes Brustkrebsrisiko definiert. Liegt die Mikronukleusrate einer Testperson somit über dem 75 %-Schwellenwert, so bedeutet dies ein erhöhtes Risiko einer Erkrankung, welches als "Odds ratio" ausgedrückt werden kann. Der Schwellenwert kann auch höher oder niedriger gewählt werden. Bei einem niedrigeren Schwellenwert erhöht sich die Sensitivität (Prozentsatz der Erkrankten, die als krank erkannt werden) während sich die Spezifität (Prozentsatz der nicht Erkrankten, für die kein Risiko erkannt wird) erniedrigt. Bei Wahl eines höheren Schwellenwertes sinkt die Sensitivität und steigt die Spezifität entsprechend.

Im Gegensatz zu den Mikronukleustests von Scott et al. (1998; 1999), die den Normalbereich der Werte als Mittelwert ± 2 Standardabweichungen definiert hatten, ist mit diesem Verfahren das Brustkrebsrisiko für alle Testindividuen sicher und reproduzierbar zu ermitteln.

### Beschreibung der Abbildungen und Tabellen

Abbildung 1: Mikronukleustest an Lymphozyten des peripheren Blutes. Gezeigt sind Beispiele von zweikernigen Lymphozyten ohne, mit einem oder zwei Mikronuklei. Die Aufnahmen erfolgten lichtmikroskopisch nach Giemsa-Färbung.

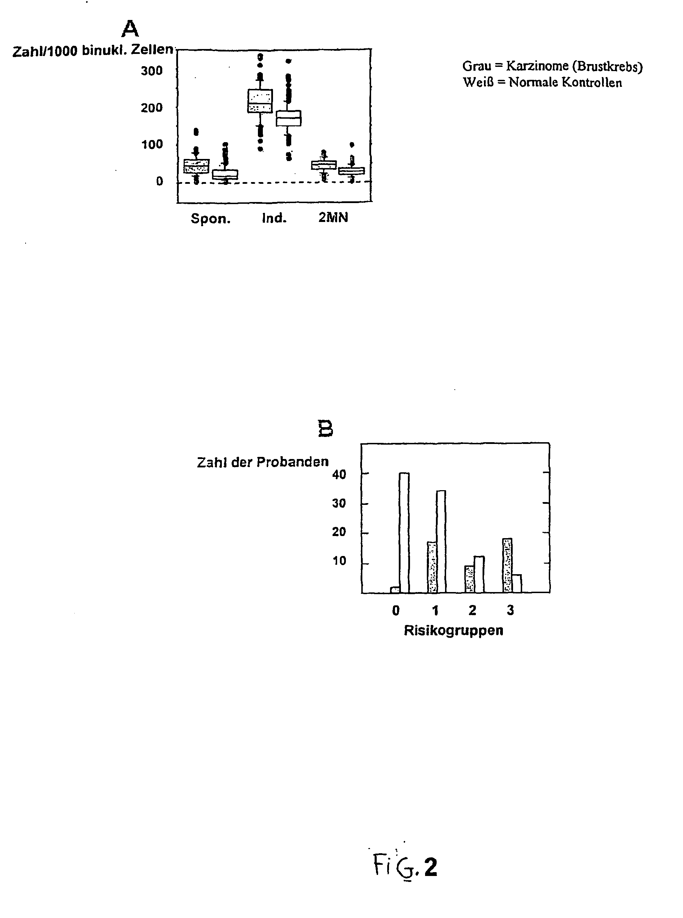

Abbildung 2A: Mikronukleus-Grundrate, induzierte Mikronukleusrate und induzierte Rate von binukleären Zellen, die zwei Mikronuklei enthalten in Brustkrebspatienten (grau) und Kontrollen (weiß). Die Darstellung erfolgt als sogenannter box-plot, wobei sich 50% der Werte innerhalb der box (des Kastens) befinden, 25% unterhalb und 25% oberhalb. Der Querstrich im Kasten entspricht dem Median. 75% der gesunden Kontrollen (weißer Kasten) liegen unterhalb der Obergrenze jeweils des weißen Kastens, womit diese dem bevorzugt gewählten Schwellenwert entspricht.

Abbildung 2B: Zahl von Patienten und Kontrollpersonen in den unterschiedlichen Risikogruppen, nach der Kombination der drei Endpunkte, die jeweils mit einem Gewicht von 1 zum Ergebnis beitrugen (Die Gruppe mit dem niedrigsten Risiko ist 0, die mit dem höchsten Risiko 3).

Tabelle 1: Beschreibende Statistik verschiedener Untergruppen von Krebspatienten und Kontrollen, die mit dem MNT in Lymphozyten des peripheren Blutes untersucht wurden. Wir bestimmten drei verschiedene Parameter: Die Rate aller MN pro 1000 Zellen nach Bestrahlung (1), die Rate pro 1000 Zellen der Zellen, die nach Bestrahlung 2 MN enthalten (2) und die MN-Rate in 1000 unbehandelten Zellen (3). Die Mittelwerte für jeden Parameter sind in Patienten mit sporadischem Brustkrebs höher im Vergleich zu Kontrollen. Die Werte bei Patienten, die eine Behandlung erhalten haben, sind höher im Vergleich zu den unbehandelten Patienten. Die Gesamtzahl und die Zahl der Patienten in den einzelnen Gruppen stimmt aufgrund fehlender Information für einige Patienten nicht immer überein (*).

Tabelle 2: Unterschiede zwischen den Mittelwerten und ihre Signifikanz für die Hauptgruppen. Die Signifikanz wurde unter Verwendung der Normal-Verteilung (t-Test) berechnet. Die Gruppe, die alle sporadischen Brustkrebspatienten umfasst, und auch die Untergruppe der nicht-behandelten sporadischen Brustkrebspatienten unterscheidet sich signifikant von den weiblichen Kontrollen in allen drei gemessenen Parametern. Es gibt einen signifikanten Unterschied in der spontanen MN-Rate zwischen behandelten und unbehandelten Brustkrebspatienten, der eine Induktion von MN durch Radiotherapie anzeigt.

Tabelle 3: "Odds ratios" für hohe und niedrige MN-Zählungen bei sporadischem Brustkrebspatienten und weiblichen Kontrollen. Es gibt fast keinen Unterschied zwischen den "Odds ratios", die für die Gruppen behandelter und unbehandelter Patienten erhalten werden. Es stellte sich heraus, dass alle "Odds ratios" größer als 5 sind, was eine hohe Wahrscheinlichkeit anzeigt, Krebspatienten mit jeder der drei bestimmten MN-Raten nachzuweisen. Ein Ergebnis, das die Information, die aus den drei MN-Raten erhalten wurde, kombiniert, kann berechnet werden und ergibt vier Gruppen. Probanden mit den niedrigsten MN-Raten wurden als Ergebnis 0 gruppiert und Probanden mit den höchsten MN-Raten als Ergebnis 3 (siehe Methoden). Ein Vergleich der Gruppen mit den niedrigsten und den höchsten Ergebnissen zeigt ein "Odds ratio" von 60, was eine hohe Sicherheit für die Kontrollen und Patienten anzeigt, aufgrund der MN-Zählung richtig gruppiert worden zu sein. Daher ist das kombinierte Ergebnis eine gute Grundlage für die Gruppierung von Probanden in Risikogruppen für die Entstehung von Brustkrebs.

### Beispiele

### Beispiel 1:

Wie für Lymphozytenkulturen üblich wird in ein heparinisiertes Röhrchen (z. B. Monovette 4,5 ml LH der Firma Sarstedt mit Lithium Heparin) Blut entnommen und bis zum Kulturansatz (maximal vier Tage) bei Zimmertemperatur aufbewahrt. Zum Ansatz der Lymphozytenkultur werden 0,3 ml des Blutes mit 3 ml Chromosomenmedium 1A (Gibco-BRL) und zwei Prozent PHA-M (Gibco BRL) zugesetzt Unmittelbar danach (innerhalb von 30 Minuten) wird die Kultur mit Gammastrahlen einer Cs-137-Strahlenquelle bestrahlt und zwar mit einer Gesamtdosis von 2 Gy. Anschließend wird die Kultur bei 37 Grad kultiviert. 44 Stunden nach der PHA-Stimulation wird Cytochalasin B (Sigma) hinzugegeben, so dass in der Kultur eine Endkonzentration von 6 µg/ml erreicht wird. Am darauffolgenden Tag wird die Kultur durch Zentrifugation der Kulturen beendet, die Zellen in hypotoner Lösung (0,56 % KCl) resuspendiert und nach nochmaliger Zentrifugation mit einer Fixierlösung bestehend aus Methanol/Eisessig (5:1) und einem gleichen Anteil 0,9 % NaCl fixiert. Die Fixierlösung wird anschließend zweimal gewechselt, wobei hier als Fixierlösung nur noch Methanol/Eisessig (5: 1) verwendet wird. In dieser Form kann die Zellsuspension längere Zeit (Wochen bis Monate) bis zur Herstellung von Präparaten aufbewahrt werden.

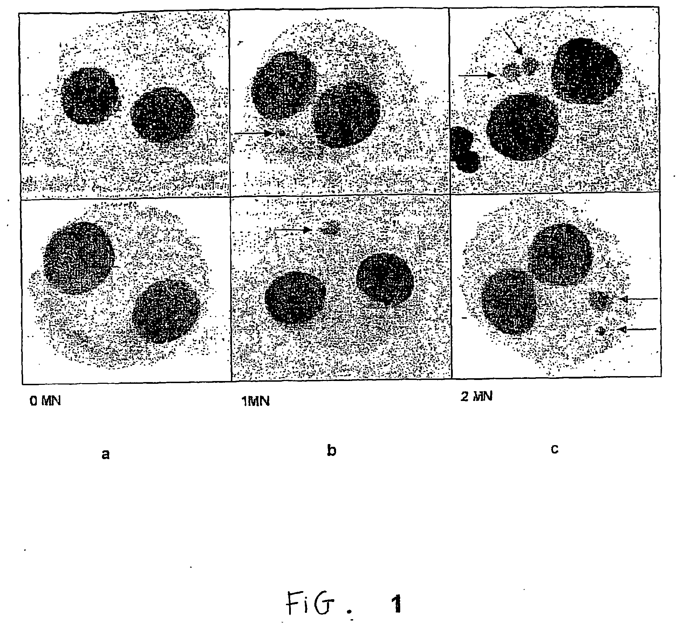

Durch Auftropfen auf Objektträger und Lufttrocknung werden aus der fixierten Zellsuspension Präparate für die Mikroskopie hergestellt. Diese Präparate werden mit 5 % (Variationsbreite 3 %-10 %) gepufferter Giemsalösung gefärbt Mit dem Mikroskop werden im Durchlicht zweikernige Zellen (Abb. 1a) aufgesucht und gezählt. Finden sich in diesen Zellen neben den beiden Zellkernen auch einer (Abb. 1b) oder mehrere (Abb. 1c) Mikronuklei deutlich von den Zellkernen separiert, so werden diese ebenfalls gezählt. Das Ergebnis lässt sich somit als Mikronukleusrate, also als die Anzahl. Mikronuklei bezogen auf 1000 zweikernige Zellen ausdrücken.

### Rechenbeispiel 1:

Es wurden drei Präparate ausgewertet, auf denen sich insgesamt 539 zweikernige Zellen gefunden haben. In diesen 539 zweikernigen Zellen wurden insgesamt 187 Mikronuklei gezählt. Hieraus ergibt sich eine Mikronukleusrate N=(1000/539)* 187=347.

Im Vergleich zu einer Kontrollgruppe, für die die Mikronukleusraten in gleicher Weise bestimmt worden waren, ergibt sich, dass die bestimmte individuelle Mikronukleusrate innerhalb der obersten 10 % der Kontrollgruppe liegt. Aus einem Vergleich der Mikronukleusraten in der Kontrollgruppe mit einer Gruppe von Probandinnen mit Brustkrebs ist bekannt, dass das Brustkrebsrisiko bei einer Mikronukleusrate innerhalb der obersten 10 % der Kontrollgruppe 8-mal höher ist (Odds Ratio = 8), als für Individuen mit Mikronukleusraten innerhalb der unteren 75% der Kontrollen.

### Beispiel 2:

Wie für Lymphozytenkulturen üblich wird in ein heparinisiertes Röhrchen (z. B. Monovette 4,5 ml LH der Firma Sarstedt mit Lithium Heparin) Blut entnommen und bis zum Kulturansatz (maximal vier Tage) bei Zimmertemperatur aufbewahrt. Zum Ansatz der Lymphozytenkultur werden 0,3 ml des Blutes mit 3 ml Chromosomenmedium 1A (Gibco-BRL) und zwei Prozent PHA-M (Gibco BRL) zugesetzt. Anschließend wird die Kultur bei 37°C kultiviert. 44 Stunden nach PHA-Stimulation wird Cytochalasin B (Sigma) hinzugegeben, so dass in der Kultur eine Endkonzentration von 6 µg/ml erreicht wird. Am darauffolgenden Tag wird die Kultur durch Zentrifugation der Kulturen beendet, die Zellen in hypotoner Lösung (0,56 % KCl) resuspendiert und nach nochmaliger Zentrifugation mit einer Fixierlösung bestehend aus Methanol/Eisessig (5:1) und einem gleichen Anteil 0,9 %ige NaCl fixiert. Die Fixierlösung wird anschließend zweimal gewechselt, wobei hier als Fixierlösung nur noch Methanol/Eisessig (5:1) verwendet wird. In dieser Form kann die Zellsuspension längere Zeit (Wochen bis Monate) bis zur Herstellung von Präparaten aufbewahrt werden.

Durch Auftropfen auf Objektträger und Lufttrocknung werden aus der fixierten Zellsuspension Präparate für die Mikroskopie hergestellt. Diese Präparate werden mit 0,1 % Acridinorange Lösung gefärbt, in zwei Küvetten mit Wasser durch kurzes Eintauchen und Schwenken differenziert und mit Wasser eingedeckt. Mit dem Fluoreszenz-Mikroskop werden bei einer Anregung von 490 nm zweikernige Zellen (Abb. 1a) aufgesucht und gezählt. Finden sich in diesen Zellen neben den beiden Zellkernen auch einer (Abb. 1b) oder mehrere (Abb. 1c) Mikronuklei deutlich von den Zellkernen separiert, so werden diese ebenfalls gezählt. Das Ergebnis lässt sich somit als Mikronukleusrate, also als Anzahl Mikronuklei bezogen auf 1000 zweikernige Zellen ausdrücken, wobei es sich in diesem Beispiel ohne Mutagen-Behandlung um die sogenannte Spontanrate handelt.

### Rechenbeispiel 2:

Es wurden zwei Präparate ausgewertet, auf denen sich insgesamt 641 binukleäre Zellen gefunden haben. In diesen 641 zweikernige Zellen wurden insgesamt 35 Mikronuklei gezählt. Hieraus ergibt sich eine Mikronukleusrate N=(1000/641)* 35 = 55.

Im Vergleich zu einer Kontrollgruppe, für die die Mikronukleusraten in gleicher Weise bestimmt worden waren ergibt sich, dass die bestimmte individuelle Mikronukleusrate innerhalb der obersten 10 % (oberhalb des Schwellenwertes) der Kontrollgruppe liegt. Aus einem Vergleich der Mikronukleusraten in der Kontrollgruppe mit einer Gruppe von Testpersonen mit Brustkrebs ist bekannt, dass das Brustkrebsrisiko bei einer Mikronukleusrate innerhalb der obersten 10 % der Kontrollgruppe 11-mal höher ist (Odds Ratio = 11), als für Patientinnen mit Mikronukleusraten innerhalb der unteren 75% der Kontrollen.

### Beispiel 3:

Wie für Lymphozytenkulturen üblich wird in ein heparinisiertes Röhrchen (z. B. Monovette 4,5 ml LH der Firma Sarstedt mit Lithium Heparin) Blut entnommen und bis zum Kulturansatz (maximal vier Tage) bei Zimmertemperatur aufbewahrt. Zum Ansatz der Lymphozytenkultur werden 0,3 ml des Blutes mit 3 ml Chromosomenmedium 1A (Gibco-BRL) angesetzt und zwei Prozent PHA-M (Gibco BRL) zugesetzt. Unmittelbar danach (innerhalb von 30 Minuten) wird die Kultur mit Gammastrahlen einer Cs-137-Strahlenquelle bestrahlt und zwar mit einer Gesamtdosis von 2 Gy. Anschließend wird die Kultur bei 37 Grad kultiviert. 44 Stunden nach PHA-Stimulation wird Cytochalasin B (Sigma) hinzugegeben, so dass in der Kultur eine Endkonzentration von 6 µg/ml erreicht wird. Am darauffolgenden Tag wird die Kultur durch Zentrifugation der Kulturen beendet, die Zellen in hypotoner Lösung (0,56 % KCl) resuspendiert und nach nochmaliger Zentrifugation mit einer Fixierlösung bestehend aus Methanol/Eisessig (5:1) und einem gleichen Anteil 0,9 % NaCl fixiert. Die Fixierlösung wird anschließend zweimal gewechselt, wobei hier als Fixierlösung nur noch Methanol/Eisessig (5:1) verwendet wird. In dieser Form kann die Zellsuspension längere Zeit (Wochen bis Monate) bis zur Herstellung von Präparaten aufbewahrt werden.

Durch Auftropfen auf Objektträger und Lufttrocknung werden aus der fixierten Zellsuspension Präparate für die Mikroskopie hergestellt. Diese Präparate werden mit 1 µg/ml DAPI Lösung in 4x SSC für 15 Minuten gefärbt. Die Eindeckung erfolgt mit 40 µl Vektashield (Fa. Vector Laboratories, Burlingame, CA) und einem Deckglas. Die Präparate werden im Fluoreszenzmikroskop der Fa. Zeiss, Deutschland, (Fluoreszenzanregung bei 413 nm) mit dem Analysesystem Metafer4 der Fa. Metasystems, Altlussheim, Deutschland, (ausgerüstet mit dem "Mikronukleus-Modul") auf zweikernige Zellen automatisch abgesucht und die Anzahl binukleärer Zellen, die Anzahl von binukleären Zellen mit 1, 2, usw. Mikronuklei erfasst. Das Ergebnis lässt sich auch als Anzahl binukleäre Zellen mit zwei Mikronuklei bezogen auf 1000 zweikernige Zellen ausdrücke.

### Rechenbeispiel 3 :

Es wurden vier Präparate ausgewertet, auf denen das Programm insgesamt 1329 binukleäre Zellen erkannt hat. Von diesen 1329 zweikernigen Zellen enthielten 78 je zwei Mikronuklei. Hieraus ergibt sich eine Rate von N=(1000/1329)* 78=59.

Im Vergleich zu einer Kontrollgruppe, für die die Rate von zwei Mikronuklei in gleicher Weise bestimmt worden war, ergibt sich, dass die beobachtete individuelle Rate von zwei Mikronuklei innerhalb der obersten 10 % der Kontrollgruppe liegt. Aus einem Vergleich der Rate von zwei Mikronuklei in der Kontrollgruppe mit einer Gruppe von Testpersonen mit Brustkrebs ist bekannt, dass das Brustkrebsrisiko bei dieser Rate von zwei Mikronuklei innerhalb der obersten 10 % der Kontrollgruppe 7-mal höher ist (Odds Ratio = 7), als für Individuen mit einer Rate von zwei Mikronuklei in den unteren 75% der Kontrollen.

### Für die drei Beispiele gemeinsam:

Handelt es sich bei den drei Beispielen um die gleiche Testperson, so lassen sich die drei Ergebnisse der Messungen zusammenfassen:
Die Testperson läge für alle drei Messungen im Bereich eines als "hoch" zu bewertenden Risikos. Bezeichnet man dies mit "h, h, h" so ergibt sich gegenüber einer Testperson mit "n, n, n" ein 60 fach höheres Risiko an Brustkrebs zu erkranken.

### Beispiel 4:

Für verschiedene Untergruppen von Brustkrebspatientinnen und Kontrollen wurde ein Mikronukleustest wie in Beispiel 1 zur Bestimmung der induzierten Mikronukleusrate und der Rate des Auftretens von 2 Mikronuklei in binukleären Zellen durchgeführt.

Die Gruppe der Brustkrebspatientinnen umfasste insgesamt 85 Probandinnen, 33 davon erhielten eine Krebstherapie, 47 nicht. In den Kontrollgruppen wurden 96 Frauen und 59 Männer untersucht.

Die Ergebnisse der Untersuchungen aus Beispiel 4 sind in den Tabellen 1 und 2 zusammengefasst. Hierin sind die einzelnen Mikronukleusraten der verschiedenen untersuchten Gruppen dargestellt. Das aus diesen Messergebnissen resultierende Brustkrebsrisiko ist in Tabelle 3 dargestellt.

Ebenso zeigt Tabelle 3, dass durch die Kombination der individuellen Mikronukleusraten die Risikoabschätzung deutlich verbessert werden kann. Die Mikronukleusraten wurden bezüglich eines hohen ("h") oder niedrigen ("n") Risikos unter Verwendung des 75 %-Schwellenwertes kodiert. Einem hohen Risiko wurde der Wert 1 zugewiesen, einem niedrigen Risiko der Wert 0. Die Addition der Werte ergab ein kombiniertes Ergebnis, das in eine der vier Risikogruppen 0, 1, 2 oder 3 eingestuft werden kann.

### Beispiel 5:

Für verschiedene Untergruppen von Testpersonen mit Brustkrebs und Kontrollen wurde ein Mikronukleustest wie in Beispiel 2 zur Bestimmung der Spontanrate für das Auftreten von Mikronuklei durchgeführt.

Die Gruppe der Brustkrebspatientinnen umfasste insgesamt 84 Probandinnen, 32 davon erhielten eine Krebstherapie, 46 nicht. In der Kontrollgruppe wurden 95 Frauen untersucht.

Die erhaltenen Mikronukleusraten der untersuchten Gruppen (Tabellen 1 und 2) wurden für die Ermittlung des Brustkrebsrisikos (Tabelle 3) herangezogen.

**Tabelle 1**

| | | Mittel ± Standardabweichung | | | |
|---|---|---|---|---|---|
| Gruppe | Zahl der Probanden * | induzierte MN (1) | Zellen mit 2 induzierten MN (2) | spontane MN (3) | Durchschnittsalter [Bereich] (Jahre) |
| Frauen | | | | | |
| Kontrollen | 96 | 175,4±3,9 | 32,6±1,5 | 26,8±2,2 | 43,3 [20-90] |
| BRCA1 Mutationsträger | 6 | 203,8±14,2 | 41,4±5,6 | 27,2±7,5 | 44,8 [32-56] |
| | | | | | |
| Brustkrebs gesamt | 85 | 210,2±5,3 | 44,5±1,8 | 48,2±3,0 | 57,3 [35-80] |
| Brustkrebs ohne Therapie | 47 | 207,0±6,5 | 44,1±2,1 | 43,1±3,6 | 59,3 [37-80] |
| Brustkrebs mit Therapie | 33 | 216,1±9,7 | 46,3±3,2 | 58,9±5,1 | 55,5 [40-74] |

**Tabelle 2**

| Gruppen | Zahl der Probanden | Mittel ± Standardabweichung | Differenz der Mittel | P-Wert |
|---|---|---|---|---|
| | | induzierte MN-Rate | | |
| Brustkrebs gesamt/ weibliche Kontrollen | 85/96 | 210,2±5,3/ 175,4±3,9 | 34,8 | < 0,0001 |
| Brustkrebs ohne Therapie/ weibliche Kontrollen | 47/ 96 | 207,0±6,5/ 175.4±3,9 | 31,5 | < 0,0001 |
| Brustkrebs mit/ ohne Therapie | 33/47 | 216,1±9,7/ 207,0±6,5 | 9,1 | 0,42 |
| | | | | |
| | | Rate der Zellen mit 2 MN | | |
| Brustkrebs gesamt/ weibliche Kontrollen | 84/ 96 | 44,5±1,8/ 32,6±1,5 | 11,9 | < 0,0001 |
| Brustkrebs ohne Therapie/ weibliche Kontrollen | 47/96 | 44,1±2,1/ 32,6±,1,5 | 11,5 | < 0,0001 |
| Brustkrebs mit/ ohne Therapie | 32/ 47 | 46,3±3,2/ 44,1±2,1 | 2,2 | 0,56 |
| | | | | |
| | | spontane MN-Rate | | |
| Brustkrebs gesamt/ weibliche Kontrollen | 84/ 95 | 48,2±3,0/ 26,8±22 | 21,5 | < 0,0001 |
| Brustkrebs ohne Therapie/ weibliche Kontrollen | 46/ 95 | 43,1±3,6/ 26,8±2,2 | 16,3 | <0,0001 |
| Brustkrebs mit/ ohne Therapie | 32/ 46 | 58,9±5,1/ 43,1±3,6 | 15,8 | 0,01 |

**Tabelle 3**

| Art der Messung | Zahl | "Odds ratio" | 95% Vertrauensintervall | P-Wert |
|---|---|---|---|---|
| induzierte MN-Rate | | | | |
| Brustkrebs gesamt/ Kontrollen | 85/ 96 | 4,9 | 2,5-9,3 | < 0,0001 |
| Brustkrebs unbehandelt/ Kontrollen | 47/ 96 | 5,3 | 2,5-11,2 | < 0,0001 |
| | | | | |
| Rate der Zellen mit 2 MN | | | | |
| Brustkrebs gesamt/ Kontrollen | 84/ 96 | 7,5 | 3,2-17,4 | < 0,0001 |
| Brustkrebs unbehandelt/ Kontrollen | 47/ 96 | 8,1 | 3,2-20,6 | < 0,0001 |
| | | | | |
| spontane MN-Rate | | | | |
| Brustkrebs gesamt/ Kontrollen | 84/ 95 | 10,3 | 4,3-24,7 | < 0,0001 |
| Brustkrebs unbehandelt/ Kontrollen | 46/ 95 | 7,7 | 2,8-21,2 | < 0,0001 |
| | | | | |
| kombiniertes Ergebnis | | | | |
| Brustkrebs unbehandelt/ Kontrollen | | | | |
| Ergebnis 0 | 42 | | | |
| Ergebnis 1 | 51 | 10,0 | 2,2-46,4 | 0,003 |
| Ergebnis 2 | 21 | 15,0 | 2,8-79,1 | 0,001 |
| Ergebnis 3 | 24 | 60,0 | 11,0-326,6 | < 0,0001 |

## Patentansprüche

1. Verfahren zur Bestimmung des Brustkrebsrisikos, folgende Schritte umfassend :
(1) Anlage einer Lymphozyten-Zellkultur aus Heparin-behandeltem peripheren Blut,
(2) mitogene Stimulation der Zellkultur sofort bis innerhalb von 6 Stunden nach Anlegen der Kultur,
(3) Zugabe eines Zytokinese-Inhibitors nach 40 bis 50 Stunden nach Anlegen der Kultur,
(4) Abbruch der Kultur nach 12 bis 24 Stunden nach Zugabe des Zytokinese-Inhibitors durch Zentrifugation und nachfolgender hypotoner Behandlung und Herstellung einer fixierten Zellsuspension,
(5) Herstellung eines zytologische Präparats,
(6) Bestimmung der Mikronukleusrate in binukleären Zellen und
(7) Risikoermittlung für das Auftreten von Brustkrebs, wobei die Risikoermittlung durch den Vergleich der Spontanrate des Auftretens von Mikronuklei in binukleären Zellen der Probandin mit der Spontanrate des Auftretens von Mikronuklei in Kontrollgruppen erfolgt.

2. Verfahren gemäß Anspruch 1, wobei die Lymphozyten bei 37 °C in Chromosommedium 1A kultiviert werden.

3. Verfahren gemäß Anspruch 1, wobei die mitogene Stimulation durch ein Mitogen ausgewählt aus der Gruppe bestehend aus Phytohämagglutinin, Lipopolysaccharid(LPS), Concanavalin A (ConA) oder Pokeweed-Mitogen (PWM) erfolgt.

4. Verfahren gemäß Anspruch 3, wobei das Phytohämagglutinin Phytohämagglutinin M (PHA-M) oder Leukoagglutinin (PHA-L) ist.

5. Verfahren gemäß Anspruch 3, wobei die mitogene Stimulation mit 2 % Phytohämagglutinin M erfolgt.

6. Verfahren gemäß Anspruch 1, wobei die Zugabe des Zytokinese-Inhibitors nach 44 Stunden und der Abbruch der Kultur nach 24 Stunden nach Zugabe des Zytokinese-Inhibitors erfolgt.

7. Verfahren gemäß Anspruch 1, wobei der Zytokinese-Inhibitor ein Cytochalasin ist.

8. Verfahren gemäß Anspruch 7, wobei das Cytochalasin Cytochalasin B ist.

9. Verfahren gemäß Anspruch 1, wobei die Herstellung des zytologischen Präparats durch Auftropfen der fixierten Zellsuspension auf einen Objektträger mit anschließender Lufttrocknung erfolgt.

10. Verfahren gemäß Anspruch 1, wobei das zytologische Präparat vor der Bestimmung der Mikronukleusrate in binukleären Zellen mit einem Farbstoff behandelt wird.

11. Verfahren gemäß Anspruch 10, wobei der Farbstoff aus der Gruppe bestehend aus Giemsa, Acridinorange, 4', 6'-Diamino-2-phenylindol (DAPI) oder Hoechst 33258 ausgewählt wird.

12. Verfahren gemäß Anspruch 1, wobei die Bestimmung der Mikronukleusrate in binukleären Zellen durch manuelles Auszählen erfolgt.

13. Verfahren gemäß Anspruch 1, wobei die Bestimmung der Mikronukleusrate in binukleären Zellen automatisch mittels Bildanalyse am Mikroskop erfolgt.

14. Verfahren gemäß Anspruch 1, wobei die Bestimmung der Mikronukleusrate in binukleären Zellen automatisch mittels Durchllusszytometrie erfolgt.

15. Verfahren gemäß der Ansprüche 1, 13 und 14, wobei die Risikoermittlung durch die Kombination der induzierten Mikronukleusrate in binukleären Zellen, der Spontanrate von Mikronuklei in binukleären Zellen und der Rate des Auftretens von zwei oder mehr Mikronuklei in binukleären Zellen der Probandin erfolgt.

## Claims

1. A method for determining the risk of breast cancer, comprising the following steps:
(1) establishing a culture of lymphocyte cells from peripheral blood treated with heparin,
(2) mitogenic stimulation of the cell culture at a time from immediately to 6 hours after establishing the culture,
(3) addition of a cytokinesis inhibitor at 40 to 50 hours after establishing the culture,
(4) termination of the culture by centrifugation and subsequent hypotonic treatment at 12 to 24 hours after addition of said cytokinesis inhibitor and preparation of a fixed cell suspension,
(5) making of a cytological preparation,
(6) determination of the micronucleus rate in binuclear cells, and
(7) determination of the risk for the occurrence of breast cancer, wherein the risk determination is carried out by comparing the rate of spontaneous occurrence of micronuclei in binuclear cells of the subject with the rate of spontaneous occurrence of micronuclei in control groups.

2. The method according to claim 1, wherein the lymphocytes are cultivated in chromosome medium 1A at 37°C.

3. The method according to claim 1, wherein the mitogenic stimulation is effected by a mitogen selected from the group consisting of phytohaemagglutinin, lipopolysaccharide (LPS), concanavalin A (ConA), or pokeweed mitogen (PWM).

4. The method according to claim 3, wherein the phytohaemagglutinin is phytohaemagglutinin M (PHA-M) or leukoagglutinin (PHA-L).

5. The method according to claim 3, wherein the mitogenic stimulation is effected by 2% phytohaemagglutinin M.

6. The method according to claim 1, wherein the addition of the cytokinesis inhibitor takes place after 44 hours and the termination of the culture takes place 24 hours after addition of the cytokinesis inhibitor.

7. The method according to claim 1, wherein the cytokinesis inhibitor is a cytochalasin.

8. The method according to claim 7, wherein the cytochalasin is cytochalasin B.

9. The method according to claim 1, wherein the making of said cytological preparation occurs by dripping said fixed cell suspension onto a slide and subsequent air-drying.

10. The method according to claim 1, wherein said cytological preparation is treated with a dye prior to determining the micronucleus rate in binuclear cells.

11. The method according to claim 10, wherein the dye is selected from the group consisting of Giemsa, acridine orange, 4',6'-diamino-2-phenylindole (DAPI), and Hoechst 33258.

12. The method according to claim 1, wherein the determination of the micronucleus rate in binuclear cells is carried out by manual counting.

13. The method according to claim 1, wherein the determination of the micronucleus rate in binuclear cells is carried out automatically by image analysis at a microscope.

14. The method according to claim 1, wherein the determination of the micronucleus rate in binuclear cells is carried out automatically by flow cytometry.

15. The method according to claims 1, 13, or 14, wherein the determination of the risk is carried out by the combination of the induced micronucleus rate in binuclear cells, the spontaneous rate of micronuclei in binuclear cells and the rate of the occurrence of two or more micronuclei in binuclear cells of the subject.

## Revendications

1. Procédé pour la détermination du risque de cancer du sein, comportant les étapes suivantes:
(1) mise en place d'une culture de cellules de lymphocytes à partir de sang périphérique traité à l'héparine,
(2) stimulation mitogène de la culture de cellules aussitôt et jusqu'à 6 heures après la mise en place de la culture,
(3) addition d'un inhibiteur de cytokinèse après 40 à 50 heures après la mise en place de la culture,
(4) arrêt de la culture après 12 à 24 heures après l'addition de l'inhibiteur de cytokinèse par centrifugation et traitement hypotonique subséquent et préparation d'une suspension de cellules fixées,
(5) élaboration d'une préparation cytologique,
(6) détermination du taux de micronucleus dans des cellules à deux noyaux et
(7) détermination du risque d'apparition de cancer du sein, tandis que la détermination du risque est effectuée par comparaison du taux spontané d'apparition de micronuclei dans les cellules à deux noyaux de la personne en test avec le taux spontané de l'apparition de micronuclei dans des groupes témoins.

2. Procédé selon la revendication 1, dans lequel les lymphocytes sont cultivés à 37°C dans un milieu chromosomique 1A.

3. Procédé selon la revendication 1, dans lequel la stimulation mitogène est effectuée au moyen d'un mitogène choisi dans le groupe consistant en phytohémaglutinine, lipopolysaccharide (LPS), concanavaline A (ConA) ou mitogène pokeweed (PWM).

4. Procédé selon la revendication 3, dans lequel la phytohémaglutinine est la phytohémagglutinine M (PHA-M) ou la leucoagglutinine (PHA-L).

5. Procédé selon la revendication 3, dans lequel la stimulation mitogène est effectuée avec 2% de phytohémagglutinine M.

6. Procédé selon la revendication 1, dans lequel l'addition de l'inhibiteur de cytokinèse s'effectue après 44 heures et l'arrêt de la culture après 24 heures après l'addition de l'inhibiteur de cytokinèse.

7. Procédé selon la revendication 1, dans lequel l'inhibiteur de cytokinèse est une cytochalasine.

8. Procédé selon la revendication 7, dans lequel la cytochalasine est la cytochalasine B.

9. Procédé selon la revendication 1, dans lequel l'élaboration de la préparation cytologique est effectuée par versage goutte à goutte de la suspension de cellules fixées sur un porte-objet avec séchage subséquent de l'air.

10. Procédé selon la revendication 1, dans lequel la préparation cytologique est traitée avec un colorant avant la détermination du taux de micronucléus dans les cellules à deux noyaux.

11. Procédé selon la revendication 10, dans lequel le colorant est choisi dans le groupe consistant en Giemsa, orange d'acridine, 4',6'-diamino-2-phénylindole (DAPI) ou Hoechst 33258.

12. Procédé selon la revendication 1, dans lequel la détermination du taux de micronucleus dans les cellules à deux noyaux est effectuée par comptage manuel.

13. Procédé selon la revendication 1, dans lequel la détermination du taux de micronucleus dans les cellules à deux noyaux est effectuée automatiquement au moyen d'une analyse d'image sur le microscope.

14. Procédé selon la revendication 1, dans lequel la détermination du taux de micronucleus dans les cellules à deux noyaux est effectuée automatiquement au moyen d'une cytométrie en flux.

15. Procédé selon les revendications 1, 13 et 14, dans lequel la détermination du risque s'effectue par la combinaison du taux de micronucléus induit dans les cellules à deux noyaux, du taux spontané de micronuclei dans les cellules à deux noyaux et du taux d'apparition de deux ou plus de deux micronucléi dans les cellules à deux noyaux de la personne en test.
